# EUROPEAN PATENT APPLICATION

(11) **EP 3 736 260 A1**
(43) Date of publication of application: **11.11.2020**
(21) Application number: 19305605.8
(22) Date of filing: 10.05.2019
(51) Int. Cl.: C07C 45/69, C07C 49/807

(54) **PROCESS FOR THE PREPARATION OF DIHALOBENZOPHENONES, NEW CHEMICALS USEFUL FOR ITS IMPLEMENTATION AND METHODS FOR PREPARING SAID CHEMICALS**

(71) Applicant: Rhodia Operations, 93300 Aubervilliers (FR)
(72) Inventor: MULLER, Eric, 69003 Lyon (FR)
(74) Representative: Valentino, Cédric

(57) **Abstract**

The invention relates to new compounds of formulae (IV): 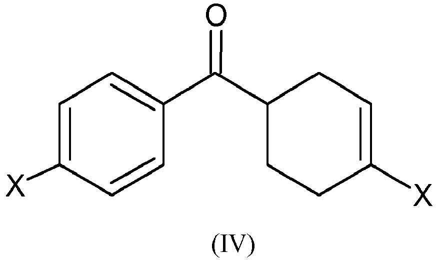 wherein X represents a halogen atom selected from the group consisting of fluorine, chlorine, bromine and iodine, said compounds being useful as precursors and/or intermediates for the preparation of 4,4'dihalobenzophenones of formula (I): 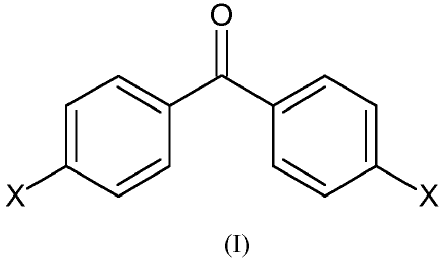 wherein X is as defined above.

## Description

### TECHNICAL FIELD

The present invention pertains to new and useful specific chemical compounds which are obtainable by cycloaddition between a 4-haloacrylophenone (also known as 4-halophenyl vinyl ketone) and a 2-halobutadiene (Diels-Alder reaction) and to their use as precursors and/or intermediates for the preparation of dihalobenzophenones.

### BACKGROUND ART

Dihalobenzophenones are valuable chemical compounds which are known to the art. They are mainly prepared by oxidation of the methylene group of a dihalodiphenyl methane to provide the corresponding dihalobenzophenone. Various other methods are known, such as those described in US 4943358, WO 2012/001131, US 5777172 and in Dunlop et al., "The preparation of 4-fluoro- and 4,4'-difluorobenzophenone", J. Am. Chem. Soc., 1933, 55(4), pp. 1665-1666. A non-exhaustive summary of other available processes for the manufacture of 4,4'-difluorobenzophenone may also be found in US 7687668.

4,4'-difluorobenzophenone (4,4'-DFBP) is the central starting material for preparing poly(aryl ether ketone)s (PAEK) which are a well-known class of engineering polymers used in various fields. These are high-performance polymers with constantly growing annual production volumes, so that the volume of 4,4'-DFBP produced worldwide annually is also included in the growth. The most important polyether ketones are the poly(ether ketone)s (PEK) and poly(ether ether ketone)s (PEEK), which feature melting points of above 330°C and high chemicals resistance.

The present invention aims at providing a new and efficient process for the preparation of dihalobenzophenones.

Another objective of the present invention is to provide a process for the preparation of dihalobenzophenones involving renewable starting materials.

### SUMMARY OF THE INVENTION

A first object of the present invention relates to a process for the preparation of a 4,4'-dihalobenzophenone of formula (I): 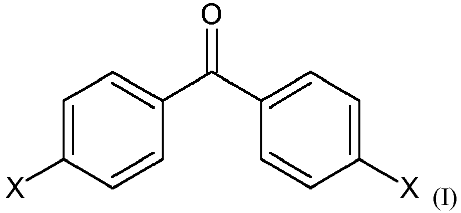 wherein X represents a halogen atom selected from the group consisting of fluorine, chlorine, bromine and iodine,
said process comprising the steps of:
a1) reacting a 2-halobutadiene of formula (II) with a 4-haloacrylophenone of formula (III): 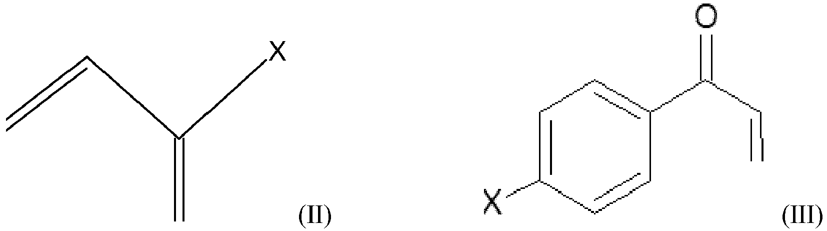 wherein X is the same as defined above,
   thereby providing a cycloadduct compound of formula (IV): 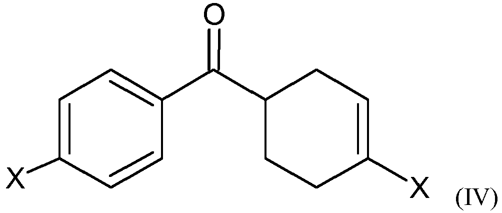 in which X is the same as defined above
a2) optionally further isolating said compound of formula (IV)
b) carrying out a dehydrogenation/aromatization of said adduct compound of formula (IV), thereby providing said 4,4'dihalobenzophenone of formula (I).

The invention also relates to the compounds of formula (IV) as defined above, which are indeed chemicals new per se.

The invention also pertains with a process for the manufacture of said compounds of formula (IV), said process involving a single Diels-Alder reaction between a 2-halobutadiene and a specific monovinylketone, i.e. a 4-haloacrylophenone.

The invention further relates to the use of a compound of formula (IV) as defined above, for the direct preparation of a 4,4'-dihalobenzophenone of formula (I), said preparation involving a dehydrogenation/aromatization reaction step on a compound of formula (IV).

According to the invention, in all its aspects, the halogen atom X is preferably selected from fluorine and chlorine.

According to the invention, in all its aspects, the Diels-Alder reaction may be carried out with or without the use of a solvent, and with or without the use of a catalyst.

According to another embodiment of the present invention, the dehydrogenation/aromatization reaction is preferably carried out in the presence of a solvent. Examples of suitable solvents are sulfoxides, preferably those chosen among dialkyl sulfoxides wherein each out of the 2 alkyl groups contains from 1 to 6 carbon atoms (especially from 1 to 4 carbon atoms), alkyl phenyl sulfoxides wherein the alkyl group contains from 1 to 6 carbon atoms (especially from 1 to 4 carbon atoms), tetrahydrothiophene 1-oxide and mixtures thereof. Good results were obtained notably when using dimethyl sulfoxide (DMSO) as the solvent.

As already mentioned, one of the advantages of the invention is to provide a process for the preparation of 4,4'-dihalobenzophenones involving renewable starting materials.

Moreover, it has been surprisingly found that the process for the preparation of compound of formula (I) according to the invention allows satisfactory conversion and selectivity.

### DETAILED DESCRIPTION OF THE INVENTION

The rational of the different chemicals (some being new per se) and chemical steps involved in the present invention may be summarized as follows: 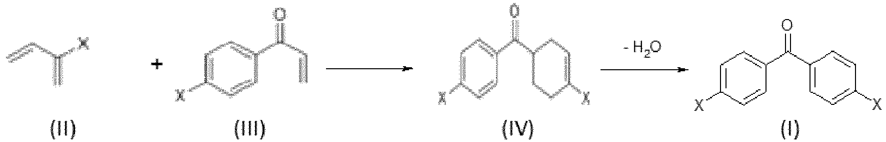 in which -H₂O symbolizes a dehydrogenation/aromatization step carried out on a cycloadduct.

Preferably, the 2-halobutadiene of formula (II) is 2-chlorobutadiene (chloroprene) or 2-fluorobutadiene (fluoroprene).

The 2-halobutadiene of formula (II) may be prepared by any conventional chemical reactions known to the one skilled in the art, or may be also commercially available products. In particular, synthesis of fluoroprene is described for example in US2469848, US2437308, US2451612 and US2437148.

Concerning the 4-haloacrylophenone of formula (III), it can be prepared for example by methylenation of the corresponding 4-haloacetophenone with formaldehyde, under conditions known to the one skilled in the art and such as described for example in J. Gen. Chem. USSR 1963, 33/5, 1512 or in Chem. Comm., 2010, 46, 1715-1717. Details for preparing 4-haloacrylophenone are also provided in the following examples.

It has been surprisingly found that a Diels-Alder reaction (step a) efficiently occurs between the 2-halobutadiene of formula (II) and the 4-haloacrylophenone of formula (III) resulting in the formation of a cycloadduct of formula (IV) as defined above. Moreover, it has been surprisingly found that said Diels-Alder reaction leads to the formation of compounds of formula (IV) with a good conversion, a low amount of by-products and a good selectivity in the desired product.

The present invention therefore also provides a process for the production of compound of formula (IV), comprising reacting a 2-halobutadiene of formula (II) with a 4-haloacrylophenone of formula (III), said two formulae being the same as defined above, and optionally further isolating compound of formula (IV) so obtained.

As already mentioned, compounds of formula (IV) are new and useful products per se, and are therefore part of the present invention.

The Diels-Alder reaction between the 2-halobutadiene of formula (II) and the 4-haloacrylophenone of formula (III) leads to the formation of two main structural isomers represented below, one of which being the compound of formula (IV):

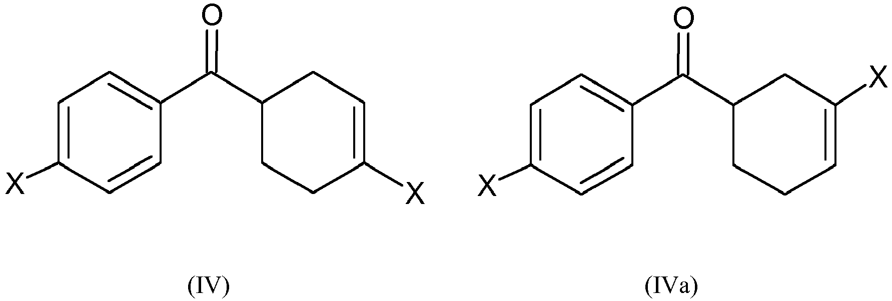

According to an embodiment of the invention, the molar ratio of compounds of formula (IV) to the compounds of formula (IVa) is of at least 50/50, preferably at least 70/30, more preferably at least 80/20, even more preferably at least 90/10.

According to an embodiment of the invention, the conversion of the Diels-Alder reaction is of at least 50%, preferably at least 70%, more preferably at least 90%.

The Diels-Alder reaction between the 2-halobutadiene of formula (II) and the 4-haloacrylophenone can be carried out under usual Diels-Alder conditions known to the person skilled in the art.

The Diels-Alder reaction may be conducted with or without a catalyst. The use of catalysts can improve kinetics and selectivity of the Diels-Alder reaction. Known Diels-Alder catalysts may be used and may include Lewis acids, such aluminium chloride, ethylaluminium dichloride, diethylaluminium chloride, trimethyl aluminium, bismuth (III) chloride, bismuth (III) trifluoromethanesulfonate, boron trifluoride, boron triacetate, cerium (III) chloride, copper (I) trifluoromethanesulfonate, copper (II) chloride, hafnium (IV) chloride, iron (II) chloride, iron (II) acetate, iron (III) chloride, iron (III) acetate, lithium perchlorate, lithium trifluoromethanesulfonate, magnesium bromide, magnesium iodide, magnesium chloride, magnesium perchlorate, scandium (III) trifluoromethanesulfonate, tin (IV) chloride, titanium (IV) chloride, titanium (IV) isopropoxide, N-trimethylsilyl-bis(trifluoromethanesulfonyl)imide, trimethylsilyl trifluoromethanesulfonate, ytterbium (III) trifluoromethanesulfonate, zinc chloride, zinc bromide, zinc iodide, zinc acetate zirconium (IV) chloride and indium (III) chloride, triphenylborane, Bronsted acids, such as inorganic mineral acids, e.g. sulfuric acid, phosphoric acid, nitric acid, hydrobromic acid or hydrochloric acid, and organic acids such as methane sulfonic acid, p-toluenesulfonic acid or carboxylic acids.

Alternatively, activated carbon, silica, alumina, silica-alumina, zirconia and zeolites may be used as such or as support for a catalytically active metal or metal compound. Suitable metals or metal compounds include alkali metals, alkaline earth metals, transition metals, noble metals, rare earth metals. The catalysts can be acidic, e.g. by treating supports with phosphoric acid, or by ion exchange of zeolites to render them into their acidic form. Examples of solid catalysts include amorphous silica-alumina, zeolites, preferably zeolites in their H-form, and acidic ion exchange resins. Other suitable catalysts that are liquids or that may be dissolved in the appropriate solvent to yield a homogeneous catalyst environment, include organic and inorganic acids, such as alkane carboxylic acid, arene carboxylic acid, sulfuric acid, phosphoric acid, hydrochloric acid, hydrobromic acid and nitric acid.

The Diels-Alder reaction may be carried out with or without a solvent. Suitable solvents may be selected from pyridine. In case a catalyst is used, it is preferred to use dichloromethane as solvent or to use no solvent at all.

The reaction may be carried out at any suitable temperature, for example from about -80 to about 120 °C, preferably from about -20 to about 100 °C, more preferably from about -10 to about 80 °C, for a time sufficient to convert the starting compounds into the desired Diels-Alder adduct, such as about 10 minutes to about 6 days, preferably about 3 hours to about 4 days, more preferably about 3 hours to about 2 days. The reaction can be carried out at ambient pressure or under increased pressure. In a preferred embodiment, the reaction is carried out at ambient pressure, such as about 1 bar, or at a pressure of up to 10 bars, preferably up to 5 bars, more preferably up to 2 bars.

According to an embodiment of the invention, the molar ratio of the 2-halobutadiene of formula (II) to the 4-haloacrylophenone of formula (III) is close to stoichiometry (namely 1/1), or above.

The process according to the invention may comprise a further step of isolation of compound of formula (IV). Suitable methods of isolation and purification are known to the person skilled in the art. For example, chromatography techniques, such as liquid chromatography, may be particularly used in the process according to the invention.

Alternatively, the mixture obtained at the end of the Diels-Alder reaction and comprising structural isomers (IV) and (IVa), and possibly by-products, can be directly used in the subsequent step of dehydrogenation/aromatization explained below (step b). In this case, a mixture comprising the following structural isomers (one of which being the desired 4,4' dihalobenzophenone of formula (I)) is obtained after completing the aromatization:

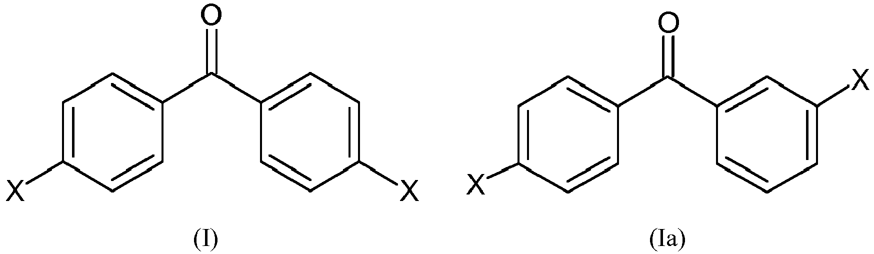

As mentioned before, in a subsequent step (step b) according to the invention, a dehydrogenation/aromatization reaction is conducted on the compound of formula (IV) in order to provide a 4,4' dihalobenzophenone of formula (I).

The reaction conditions for aromatization of the compound of formula (IV) are known to a person skilled in the art.

Suitable catalysts or reagents to conduct the aromatization reaction may be (i) a copper salt or a copper oxide in presence of a base (such as an alkali metal acetate or alkali metal hydroxide), or (ii) manganese dioxide.

Suitable solvents for the aromatization reaction may be for example chosen from sulfoxides such as DMSO (especially when the catalyst or reagent used to conduct the aromatization reaction is a copper salt or a copper oxide in presence of a base), and from chlorobenzene and toluene (especially when the catalyst or reagent used to conduct the aromatization reaction is manganese dioxide).

The aromatization reaction may be carried out at any suitable temperature, for example from about -10 to about 120 °C, preferably from about -5 to about 80 °C, more preferably from about 0 to about 40 °C.

The present invention also relates to a process for the direct preparation of a 4,4'-dihalobenzophenone of formula (I): 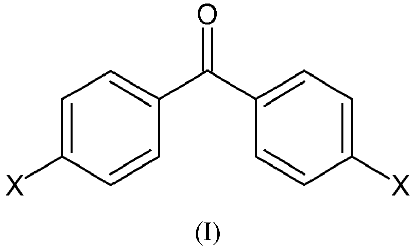 wherein X represents a halogen atom selected from the group consisting of fluorine, chlorine, bromine and iodine, comprising carrying out the dehydrogenation/aromatization of the compound of formula (IV) as defined above. Preferably, X is selected from fluorine and chlorine.

Another object of the invention relates to a process for producing a 4,4'-dihalobenzophenone of formula (I): 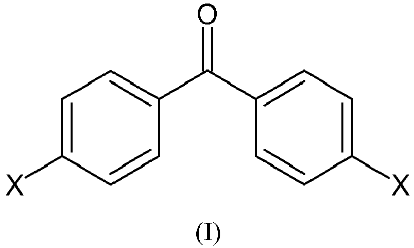 wherein X represents a halogen atom selected from the group consisting of fluorine, chlorine, bromine and iodine, comprising:
a) providing a compound of formula (IV) as defined above,
b) carrying out the dehydrogenation/aromatization of said compound of formula (IV).

When 4,4'-difluorobenzophenone is desired, it may be prepared by effecting a halogen-fluorine exchanging reaction between compound of formula (I) wherein X is chlorine, bromine or iodine, and an alkali fluoride, such as potassium fluoride, as described in US 4,453,009 and US2002161227. Alternatively, it may also be possible to proceed with said halogen-fluorine exchanging reaction directly on the compound of formula (IV) wherein X is chlorine, bromine or iodine, in accordance with the teaching of US4792618.

According to an embodiment of the invention, 4,4'-dihalobenzophenone of formula (I) is further isolated by separation methods known to the person skilled in the art. For example, recrystallization may be carried out according to the method described in document US 4,873,372.

Poly(aryl ether ketone)s (PAEK) are a well known class of engineering polymers useful in various fields of scientific and commercial endeavour. PAEK polymers are generally prepared by aromatic nucleophilic substitution of both the halogenides of a 4,4'-dihalobenzophenone (especially, by aromatic nucleophilic substitution of both the fluorides of 4,4'-difluorobenzophenone) by a nucleophilic oxygen atom from a co-monomer. For example, p-hydroquinone, commonly referred to as "hydroquinone" and/or at least one bisphenol (such as bisphenol A, bisphenol S, bisphenol O, 4,4'-dihydroxybiphenyl and mixtures thereof) can be used as the co-monomer; the hydrogen atom from each of the two aromatic hydroxyl groups of p-hydroquinone and/or of the bisphenol is advantageously deprotonated with at least one base (such as NaOH, Na₂CO₃, K₂CO₃ and mixtures thereof) to form a nucleophile which reacts with the 4,4'-dihalobenzophenone (preferably, 4,4'-difluorobenzophenone) to form a PAEK polymer via a nucleophilic substitution mechanism, with the halogen atoms of the 4,4'-dihalobenzophenone acting as leaving groups. Processes for preparing PAEK polymers, including those using a 4,4'-dihalobenzophenone such as 4,4'-difluorobenzophenone, can be found notably in U.S. Pat. Nos. 3,953,400, 3,956,240, 3,928,295, and 4,176,222, all incorporated herein by reference.

Then, facets of the invention relate to :
- the use of a 4,4'-dihalobenzophenone of formula (I) prepared from a compound of formula (IV) as previously defined for the manufacture of a poly(aryl ether ketone), in particular poly(ether ether ketone),
- the use of a compound of formula (IV) as previously defined for the manufacture of a poly(aryl ether ketone), in particular poly(ether ether ketone), typically with a 4,4'-dihalobenzophenone of formula (I) as synthesis intermediate, and
- the use of a 4,4'-dihalobenzophenone of formula (I) as synthesis intermediate in the manufacture of a poly(aryl ether ketone) [in particular poly(ether ether ketone] from a compound of formula (IV) as above defined.

Precisely an object of the present invention concerns a process for the manufacture of a (poly aryl ether ketone), said process comprising the following steps:
i) providing a compound of formula (IV) as defined above,
ii) carrying out the dehydrogenation/aromatization of said compound of formula (IV) to prepare a 4,4'-dihalobenzophenone of formula (I) as defined above,
iii) optionally, isolating the 4,4'-dihalobenzophenone,
iv) optionally, effecting a halogen-fluorine exchanging reaction between the 4,4'-dihalobenzophenone and an alkali fluoride as explained above ,
v) carrying out the polycondensation of the 4,4'-dihalobenzophenone with at least one aromatic compound comprising 2 hydroxyl groups, in particular with hydroquinone and/or at least one bisphenol, preferably in the presence of at least one base.

Should the disclosure of any patents, patent applications, and publications which are incorporated herein by reference conflict with the description of the present application to the extent that it may render a term unclear, the present description shall take precedence.

The present invention will now be illustrated by the following examples, which are not intended to be limiting.

### EXAMPLE

This example illustrates the invention for the preparation of dihalobenzophenones (compounds of formula (I)) from chloroprene (compound of formula (II)) and a 4-chloroacrylophenone (compound of formula (III)

### 1/ Preparation of the 4-chloroacrylophenone

### Step 1 : preparation of a (methyl)(chlorocyclohexenyl) ketone

In a carousel tube fitted with a PTFE septum screw cap methylvinylketone (1.0 g, 14.3 mmol) was weighted. Then chloroprene (1.26 g, 14.3 mmol) and 1.2 mL of dichloromethane (DCM) were added. After cooling at 0°C, methanesulfonic acid (288 mg, 3.0 mmol) was added. The mixture was stirred 2h at 0°C and 1h at room temperature. The product is purified by filtration over a pad of silica gel washed with DCM. After evaporation of DCM *in vacuo* we obtained 1.20 g (yield 53%) of the cycloadduct **1-Cl**, which is (methyl)(chlorohexenyl) ketone as a 90/10 mixture of para/meta isomers :

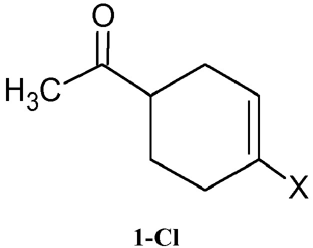

### Step 2 : aromatization of the cycloadduct (methyl)(chlorohexenyl) ketone

In a 50-ml round bottom flask fitted with a Dean-Stark trap and reflux condenser was charged 10g of Manganese dioxide (activated, Aldrich), 20 mL of toluene and 1.01 of cycloadducts **1-Cl** prepared in step 1. The mixture was refluxed for 4h. After cooling and filtration, GC titration of the toluene solution indicated the formation of 267 mg (yield 27%) of chloroacetophenones, as a 90/10 mixture of para/meta isomers.

### Step 3 : Alpha-methylenation of the chloroacetophenone

In a 2 liter reactor under argon atmosphere filed with 900 mL of DMSO is added : 21.6 g of glycine hydrochloride (0.194 mol), 87.4 g of paraformaldehyde (2.91 mol), 150 g of 4'-chloroacetophenone (0.97 mol) and 725 mg of hydroquinone. The mixture is heated at 80°C for 90 minutes with mechanical stirring. After cooling to room temperature, the product is extracted five times with 900 mL of diethylether. The combined ether layers are evaporated to 1 liter, washed five times with 450 mL of water, dried over MgSO4 and evaporated *in vacuo* to afford 116.2g of a pale yellow oil. RMN and GC analysis indicated a purity in 4'-chloroacrylophenone of 89% corresponding to a yield of 64%.

### 2/ Diels-Alder reaction between chloroacrylophenone and chloroprene

In a carousel tube fitted with a PTFE septum screw cap chloroprene (796 mg, 9.0 mmol) was weighted. Then DCM (1mL) and 4'-chloroacrylophenone (1.0 g, 6.0 mmol) were added. After cooling at 0°C, methanesulfonic acid (288 mg, 3.0 mmol) was added. The reaction mixture was stirred at 0°C during 1 h. After filtration on silica gel in DCM and evaporation *in vacuo* we obtained 1.2 g of a pale yellow oil. NMR and GC titrations indicated a purity of 91% in cycloadducts **2-Cl** (yield 71%) and a 4,4'/3,4' ratio of 91/9. 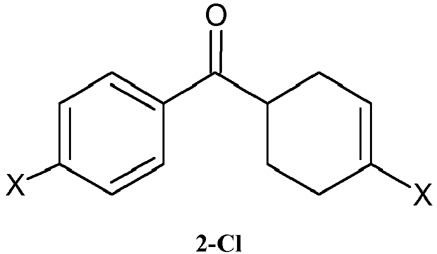 MS : 254
¹H and [¹³C] NMR :

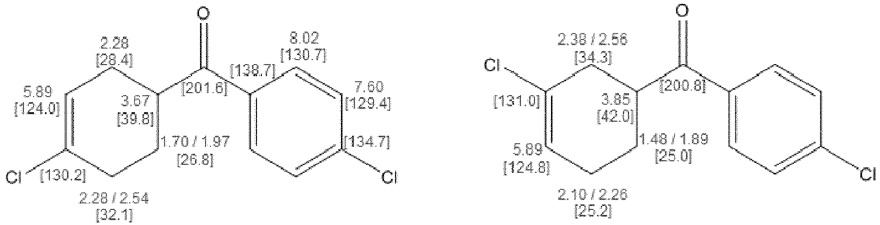

### 3/ Aromatisation of the 2-Cl cycloadducts

### 3.1 Aromatization of 2-Cl with MnO2

In a 50-ml round bottom flask fitted with a Dean-Stark trap and reflux condenser was charged 6.2 g of Manganese dioxide (activated, Aldrich), 12 mL of toluene and 0.85 g of cycloadduct **2-Cl** (para/meta isomers mixture). The reaction media was refluxed for 6h. GC titration of the toluene solution indicated the formation of 305 mg (yield 36%) of 4,4' and 3,4'-dichlorobenzophenones.

### 3.2 Aromatization of 2-Cl with Cu(OAc)₂

In a carousel tube, para/meta mixture of cyloadduct **2-Cl** (155 mg, 0.61 mmol) and copper II acetate (54 mg, 0.3mmol) was weighted. Then DMSO (2 mL) and sodium acetate (164 mg, 2 mmol) were added. The reaction mixture was stirred 20h at 90°C under air atmosphere (open flask). GC titration of the final reaction media indicated the near complete conversion of **2-Cl** and the formation of 93 mg (61% yield) of dichlorobenzophenones (3,4'/4,4' mixture).

## Claims

1. Process for the preparation of a 4,4'-dihalobenzophenone of formula (I): 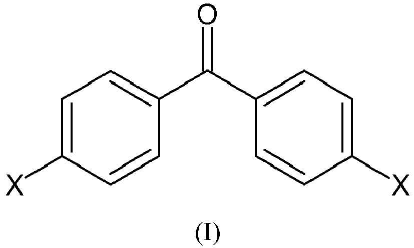 wherein X represents a halogen atom selected from the group consisting of fluorine, chlorine, bromine and iodine,
said process comprising the steps of:
a1) reacting a 2-halobutadiene of formula (II) with a 4-haloacrylophenone of formula (III): 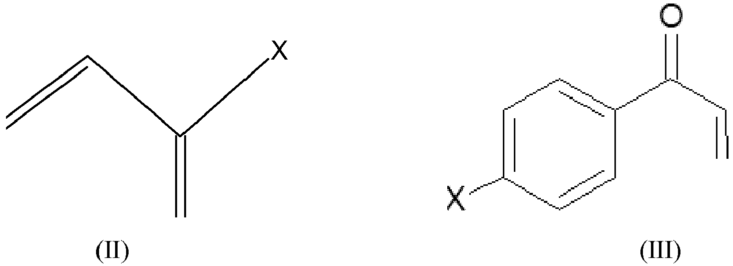 wherein X is the same as defined above,
thereby providing a cycloadduct compound of formula (IV): 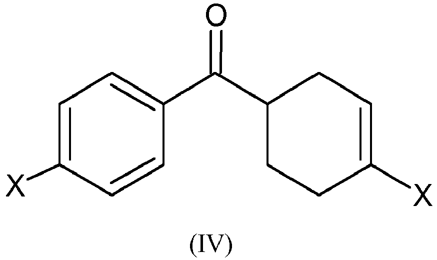 in which X is the same as defined above
a2) optionally further isolating said compound of formula (IV)
b) carrying out a dehydrogenation/aromatization of said adduct compound of formula (IV), thereby providing said 4,4' dihalobenzophenone.

2. Process according to claim 1, wherein the dehydrogenation/aromatization is carried out in the presence of a solvent.

3. Compound of formula (IV): 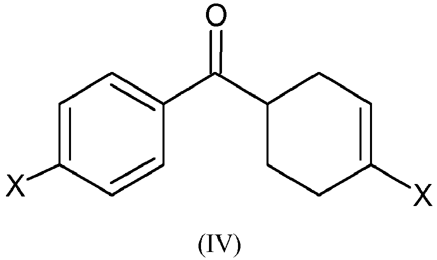 wherein X represents a halogen atom selected from the group consisting of fluorine, chlorine, bromine and iodine.

4. Compound according to claim 3, wherein the halogen atom is selected from fluorine and chlorine.

5. Process for the preparation of a compound of formula (IV) as defined in any of claims 3 or 4, comprising reacting a 2-halobutadiene of formula (II) as defined above with a 4-haloacrylophenone of formula (III), and optionally further isolating compound of formula (IV).

6. Process according to claim 5, wherein the molar ratio of the 2-halobutadiene of formula (II) to the 4-haloacrylophenone of formula (III) is of at least 1/1.

7. Process for the preparation of a 4,4'-dihalobenzophenone of formula (I): 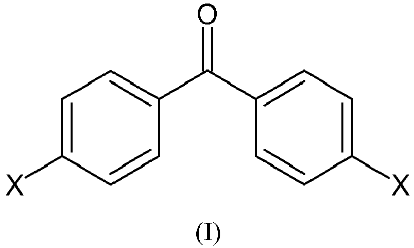 wherein X represents a halogen atom selected from the group consisting of fluorine, chlorine, bromine and iodine,
comprising carrying out the dehydrogenation/aromatization of a compound of formula (IV): 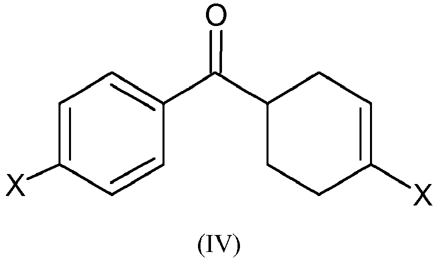 wherein X is as defined above.

8. Process for the preparation of a 4,4'-dihalobenzophenone of formula (I): 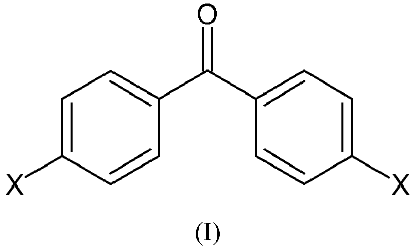 wherein X represents a halogen atom selected from the group consisting of fluorine, chlorine, bromine and iodine,
comprising:
a) providing a compound of formula (IV): 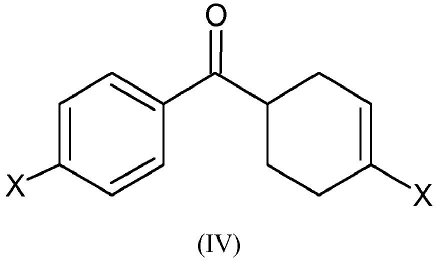 wherein X is as defined above,
b) carrying out the dehydrogenation/aromatization of said compound of formula (IV),
c) optionally further isolating compound of formula (I).

9. Use of a compound of formula (IV) as defined in claim 3 for the manufacture of a poly(aryl ether ketone), in particular poly(ether ether ketone).
